# EUROPEAN PATENT APPLICATION

(11) **EP 4 611 385 A1**
(43) Date of publication of application: **03.09.2025**
(21) Application number: 23882427.0
(22) Date of filing: 12.10.2023
(51) Int. Cl.: H04N 23/611, A61B 5/00, A61B 5/107, A61B 5/16, H04N 23/60, H04N 23/661

(54) **DIAGNOSIS SYSTEM, DIAGNOSIS DEVICE, PROGRAM, DIAGNOSIS METHOD, METHOD FOR DIAGNOSING SKIN, AND METHOD FOR DIAGNOSING STRESSES**

(30) Priority: 26.10.2022 JP 2022171404
(71) Applicant: Shiseido Company, Ltd., Chuo-ku Tokyo 104-0061 (JP)
(72) Inventor: TAKEHI, Go, Tokyo 104-0061 (JP); TAKATA, Motoki, Tokyo 104-0061 (JP); CHEN, Yi, Tokyo 104-0061 (JP); KOBAYASHI, Yohei, Tokyo 104-0061 (JP); SEKINO, Megumi, Tokyo 104-0061 (JP); LI, Juncheng, Tokyo 104-0061 (JP)
(74) Representative: Ter Meer Steinmeister & Partner
(86) International application number: PCT/JP2023/036984
(87) International publication number: WO 2024/090218

(57) **Abstract**

It is possible to efficiently diagnose when necessary requirements for each diagnosis are different. A diagnosis system according to an embodiment of the present invention is a system that includes a terminal and a server. The system includes: a first diagnosis processing unit configured to perform a first diagnosis process; a second diagnosis processing unit configured to perform a second diagnosis process different from the first diagnosis process; a facial state detection unit configured to detect a facial state of a user; and a diagnosis process switching unit configured to determine which of the first diagnosis process or the second diagnosis process is to be performed according to a result of the detecting.

## Description

### TECHNICAL FIELD

The present invention relates to a diagnosis system, a diagnosis device, a program, a diagnosis method, a skin diagnosis method, and a stress diagnosis method.

### BACKGROUND OF THE INVENTION

Conventionally, a method of diagnosing a user's condition by analyzing an image of the user's face is known. For example, Patent Document 1 describes acquiring a plurality of images representing the process of facial expression change, extracting images between the image at the start of the facial expression change and the image at the end of the facial expression change as an analysis target, and analyzing skin features using the extracted images.

### RELATED-ART DOCUMENT

### Patent Document

Patent Document 1: Japanese Unexamined Patent Application Publication No. 2019-098168

### SUMMARY OF THE INVENTION

### Problems to be Solved by the Invention

The imaging method and the facial state of the user required for one diagnosis are different from those required for another diagnosis. Therefore, when multiple types of diagnoses are performed, necessary requirements required for each diagnosis must be fulfilled, and there are many diagnostic steps, placing a burden on the user.

Therefore, an object of the present invention is to efficiently diagnose when necessary requirements for each diagnosis are different.

### Means for Solving the Problems

In an embodiment of the present invention, a diagnosis system includes a terminal and a server. The system includes: a first diagnosis processing unit configured to perform a first diagnosis process; a second diagnosis processing unit configured to perform a second diagnosis process different from the first diagnosis process; a facial state detection unit configured to detect a facial state of a user; and a diagnosis process switching unit configured to determine which of the first diagnosis process or the second diagnosis process is to be performed according to a result of the detecting.

### Effects of the Invention

According to the present invention, it is possible to efficiently diagnose when necessary requirements for each diagnosis are different.

### BRIEF DESCRIPTION OF THE DRAWINGS

[FIG. 1] FIG. 1 is a diagram for explaining a flow of process when multiple types of diagnoses are performed sequentially.
[FIG. 2] FIG. 2 is a diagram for explaining an outline of the present invention.
[FIG. 3] FIG. 3 is an overall configuration example according to an embodiment of the present invention.
[FIG. 4] FIG. 4 is a functional block diagram of a diagnosis device according to an embodiment of the present invention.
[FIG. 5] FIG. 5 is a flowchart of a diagnosis process according to an embodiment of the present invention.
[FIG. 6] FIG. 6 is a hardware configuration diagram of the diagnosis device and a terminal according to an embodiment of the present invention.

### DETAILED DESCRIPTION OF THE INVENTION

Hereinafter, an embodiment of the present invention will be described based on the drawings.

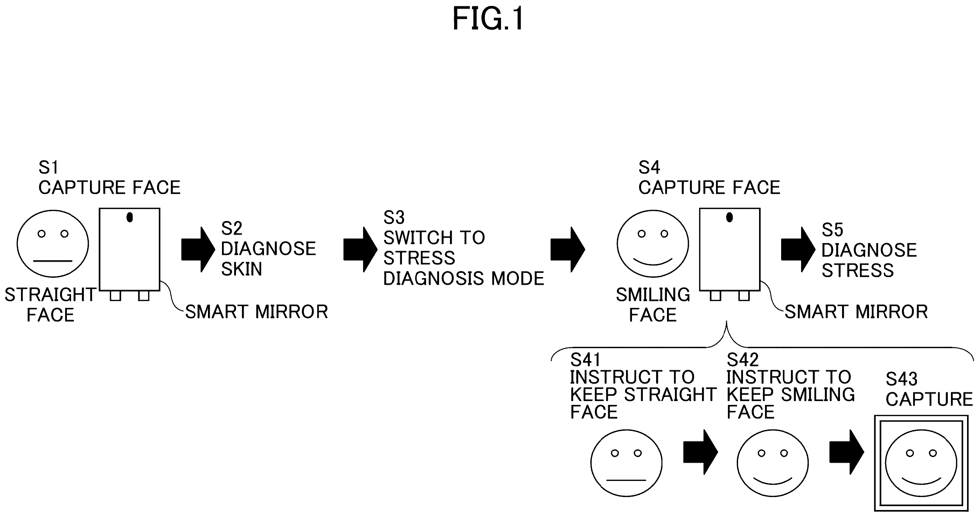

First, with reference to FIG. 1, a flow of processes when multiple types of diagnoses (in the example of FIG. 1, two diagnoses: a skin diagnosis and a stress diagnosis) are sequentially performed will be described. In steps 1 and 2, a skin diagnosis process is performed, and in steps 4 and 5, a stress diagnosis process is performed.

In step 1 (S1), a device having an imaging function such as a smart mirror captures a user's face when the user has a straight face. In order to perform the skin diagnosis, a high-resolution image of the user's straight face is required.

In step 2 (S2), the skin of the user is diagnosed using the image captured in S1.

In step 3 (S3), a skin diagnosis mode of S1 and S2 is switched to a stress diagnosis mode.

In step 4 (S4), the smart mirror or the like captures the user's face when the user has a smiling face. In order to perform the stress diagnosis, a low-resolution image of the user's smiling face is required. Hereinafter, S4 will be described in detail in steps 41 to 43.

In step 41 (S41), the smart mirror or the like instructs the user to keep a straight face.

In step 42 (S42), the smart mirror or the like instructs the user to keep a smiling face.

In step 43 (S43), the smart mirror or the like captures the user.

In step 5 (S5), stress of the user is diagnosed using the image captured in S4.

As described above, when multiple types of diagnoses (in the example of FIG. 1, two diagnoses: the skin diagnosis and the stress diagnosis) are performed, the necessary requirements (in the example of FIG. 1, the skin diagnosis requires a high-resolution image of a straight face, and the stress diagnosis requires a low-resolution image of a smiling face) required for each diagnosis must be fulfilled, and there are many diagnostic steps, placing a burden on the user. Therefore, in the present invention, the diagnosis processes are switched according to the facial state of the user.

### <Outline>

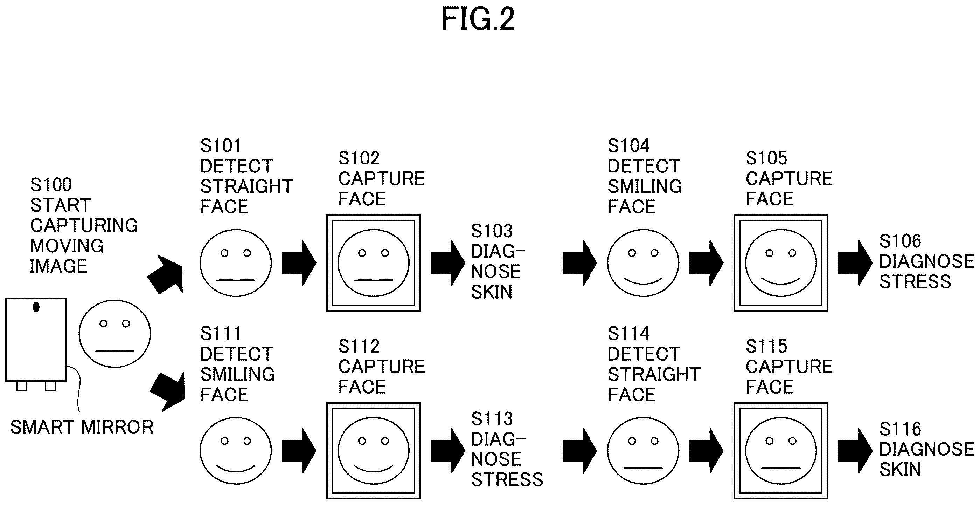

FIG. 2 is a diagram for explaining an outline of the present invention.

In step 100 (S100), a device having an imaging function such as a smart mirror starts capturing a moving image of the user's face. When a straight face is detected during capturing of the moving image, the process proceeds to step 101, and when a smiling face is detected during capturing of the moving image, the process proceeds to step 111.

First, steps 101 to 106 will be described.

In step 101 (S101), it is assumed that a straight face of the user is detected during capturing of the moving image started in S100.

In step 102 (S102), in response to the detection of the straight face of the user in S101, the smart mirror or the like switches to a high-resolution capturing mode and captures the user's face (captures a still image).

In step 103 (S103), the skin of the user is diagnosed using the image captured in S102. Thereafter, the smart mirror or the like resumes capturing of the moving image of the user's face.

In step 104 (S104), it is assumed that a smiling face of the user is detected during capturing of the moving image resumed in S103.

In step 105 (S105), in response to the detection of the smiling face of the user in S104, the smart mirror or the like switches to a low-resolution capturing mode and captures the user's face (captures a still image).

In step 106 (S106), the stress of the user is diagnosed using the image captured in S105.

Next, steps 111 to 116 will be described.

In step 111 (S111), it is assumed that a smiling face of the user is detected during capturing of the moving image started in S100.

In step 112 (S112), in response to the detection of the smiling face of the user in S111, the smart mirror or the like switches to the low-resolution capturing mode and captures the user's face (captures a still image).

In step 113 (S113), the stress of the user is diagnosed using the image captured in S112. Thereafter, the smart mirror or the like resumes capturing of the moving image of the user's face.

In step 114 (S114), it is assumed that a straight face of the user is detected during capturing of the moving image resumed in S113.

In step 115 (S115), in response to the detection of the straight face of the user in S114, the smart mirror or the like switches to the high-resolution capturing mode and captures the user's face (captures a still image).

In step 116 (S116), the skin of the user is diagnosed using the image captured in S115.

### <Overall Configuration Example>

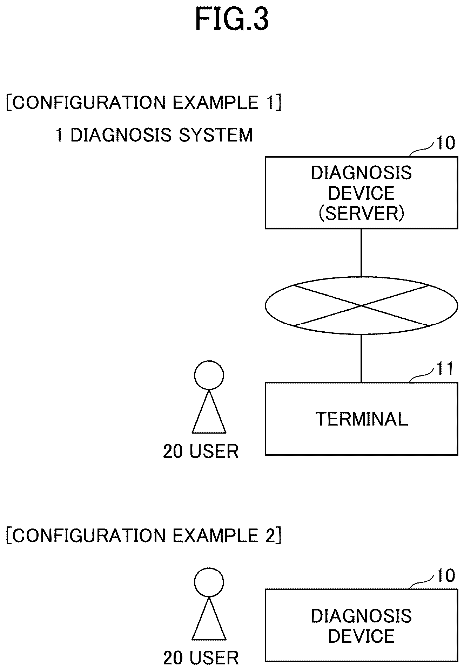

FIG. 3 is an overall configuration example according to an embodiment of the present invention.

### [Configuration Example 1]

A diagnosis system 1 includes a diagnosis device (for example, a server) 10 and a terminal 11. The diagnosis device 10 and the terminal 11 are communicably connected via an arbitrary network. In Configuration Example 1, a user 20 (or a beauty advisor at a cosmetics store) operates the terminal 11. Each of them will be described below.

### <<Diagnosis Device>>

The diagnosis device 10 determines which of a plurality of diagnosis processes (a first diagnosis process and a second diagnosis process different from the first diagnosis process) is to be performed according to the facial state of the user 20, and performs the diagnosis process. For example, the diagnosis device 10 may be a server or the like consisting of one or more computers.

For example, the diagnosis device 10 detects the facial expression of the user 20 based on the features of the image in which the face of the user 20 is captured. The diagnosis device 10 causes the terminal 11 to capture a still image with high quality when the facial expression of the user 20 is a straight face, and causes the terminal 11 to capture a still image with low quality when the facial expression of the user 20 is a smiling face.

### <<Terminal>>

The terminal 11 captures the face of the user 20 and transmits the captured image to the diagnosis device 10. The terminal 11 also receives and displays the diagnosis result by the diagnosis device 10. The terminal 11 may perform a part of the processes of the diagnosis device 10 described in the present specification. For example, the terminal 11 may be a smart mirror (specifically, a display having an imaging function) installed in a store, a smartphone having an imaging function, a tablet having an imaging function, or the like. The terminal 11 that does not have an imaging function may acquire an image by having an imaging device capture an image.

### [Configuration Example 2]

In Configuration Example 2, the user 20 (or a beauty advisor at a cosmetics store) operates the diagnosis device 10.

### <<Diagnosis device>>

The diagnosis device 10 determines which of a plurality of diagnosis processes (a first diagnosis process and a second diagnosis process different from the first diagnosis process) is to be performed according to the facial state of the user 20, and performs the diagnosis process. For example, the diagnosis device 10 may be a smart mirror (specifically, a display having an imaging function), a smartphone having an imaging function, a tablet having an imaging function, or the like. The diagnosis device 10 that does not have an imaging function may acquire an image by having an imaging device capture an image.

For example, the diagnosis device 10 captures the face of the user 20 and detects the facial expression of the user 20 based on the features of the image in which the face of the user 20 is captured. The diagnosis device 10 captures a still image with high quality when the facial expression of the user 20 is a straight face, and captures a still image with low quality when the facial expression of the user 20 is a smiling face, and displays the diagnosis result.

### <Functional Block>

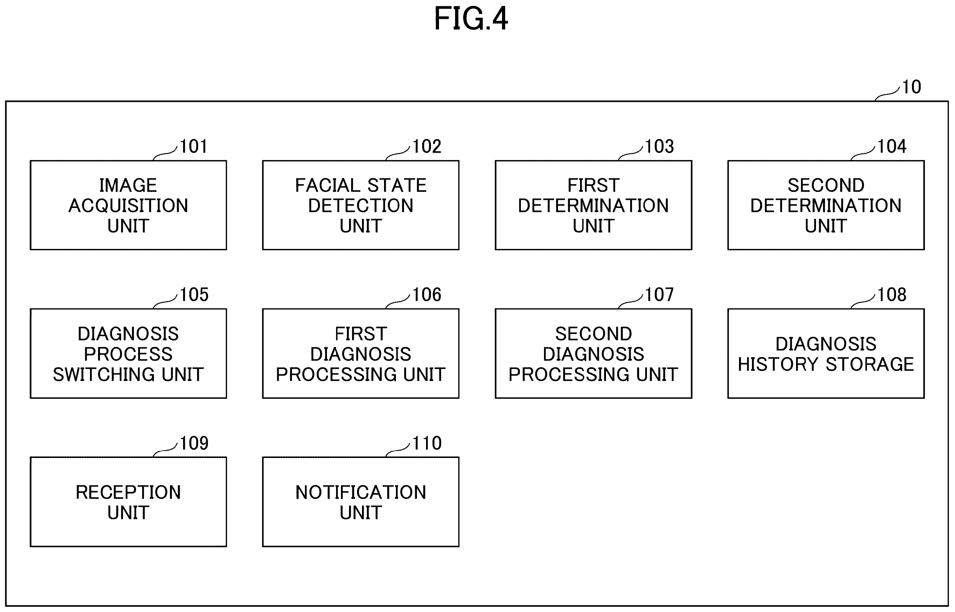

FIG. 4 is a functional block diagram of the diagnosis device 10 according to an embodiment of the present invention. The diagnosis device 10 includes an image acquisition unit 101, a facial state detection unit 102, a first determination unit 103, a second determination unit 104, a diagnosis process switching unit 105, a first diagnosis processing unit 106, a second diagnosis processing unit 107, a diagnosis history storage 108, a reception unit 109, and a notification unit 110. The diagnosis device 10 functions as the image acquisition unit 101, the facial state detection unit 102, the first determination unit 103, the second determination unit 104, the diagnosis process switching unit 105, the first diagnosis processing unit 106, the second diagnosis processing unit 107, the reception unit 109, and the notification unit 110, by executing a program.

The image acquisition unit 101 acquires an image in which the face of the user 20 is captured. For example, the image acquisition unit 101 acquires a moving image captured when the image capture mode is "moving image capture mode". The image acquisition unit 101 acquires a still image of high quality captured when the image capture mode is "skin diagnosis mode". The image acquisition unit 101 acquires a still image of low quality captured when the image capture mode is "stress diagnosis mode".

The facial state detection unit 102 detects the facial state of the user 20.

For example, the facial state detection unit 102 detects the facial expression of the user 20 based on the features of the image acquired by the image acquisition unit 101. Specifically, the facial state detection unit 102 determines whether the user 20 has a straight face based on the coordinates of each part (for example, eyes, nose, mouth, and the like) of the face of the user 20 in the moving image captured when the image capture mode is "moving image capture mode". The facial state detection unit 102 determines whether the user 20 has a smiling face based on the coordinates of each part (for example, eyes, nose, mouth, and the like) of the face of the user 20 in the moving image captured when the image capture mode is "moving image capture mode".

The first determination unit 103 determines whether the first diagnosis process has been performed a predetermined number of times (at least one time). For example, the first determination unit 103 may determine whether the first diagnosis process has been performed a predetermined number of times in the session (that is, whether it has been performed in the current sequence of processes). For example, the first determination unit 103 may determine whether the first diagnosis process has been performed a predetermined number of times outside the session (for example, whether it has been performed on another day in the past). (The first determination unit 103 may determine whether the user 20 has performed the first diagnosis process outside the session by specifying the user 20 by face recognition, ID input, or the like.)

The second determination unit 104 determines whether the second diagnosis process has been performed a predetermined number of times (at least one time). For example, the second determination unit 104 may determine whether the second diagnosis process has been performed a predetermined number of times in the session (that is, whether it has been performed in the current sequence of processes). For example, the second determination unit 104 may determine whether the second diagnosis process has been performed a predetermined number of times outside the session (for example, whether it has been performed on another day in the past). (The second determination unit 104 may determine whether the user 20 has performed the second diagnosis process outside the session by specifying the user 20 by face recognition, ID input, or the like.)

The diagnosis process switching unit 105 determines which of the first diagnosis process or the second diagnosis process is to be performed according to the result (for example, the facial expression of the user 20) detected by the facial state detection unit 102.

The first diagnosis processing unit 106 performs the first diagnosis process. The first diagnosis processing unit 106 may perform the first diagnosis process when the first determination unit 103 determines that the first diagnosis process has not yet been performed a predetermined number of times. For example, a representative value such as an average value of the results of a plurality of diagnoses may be used, or a part of the results of a plurality of diagnoses may be used. The predetermined number of times may be set by the user 20.

The second diagnosis processing unit 107 performs the second diagnosis process different from the first diagnosis process. The second diagnosis processing unit 107 may perform the second diagnosis process when the second determination unit 104 determines that the second diagnosis process has not yet been performed a predetermined number of times. For example, a representative value such as an average value of the results of a plurality of diagnoses may be used, or a part of the results of a plurality of diagnoses may be used. The predetermined number of times may be set by the user 20.

Only the first diagnosis process, only the second diagnosis process, or both of the first diagnosis process and the second diagnosis process may be selected by the user 20, and the first diagnosis processing unit 106 and the second diagnosis processing unit 107 may perform the diagnosis process selected by the user 20.

The diagnosis history storage 108 stores the history in which the first diagnosis process is performed and the history in which the second diagnosis process is performed. An expiration date may be set for the information of each history, and the first determination unit 103, the first diagnosis processing unit 106, the second determination unit 104, and the second diagnosis processing unit 107 may use only the information of the history within the expiration date (the expiration date may be set manually or automatically based on past data (for example, changes in individual diagnosis results (changes in stress status)), season, and the like).

The reception unit 109 receives an instruction (for example, setting) input by the user 20 into the terminal 11 (in the case of Configuration Example 1) or into the diagnosis device 10 (in the case of Configuration Example 2).

The notification unit 110 notifies the user 20 of the information (for example, results of a diagnosis) by displaying the information on the screen of the terminal 11 (in the case of Configuration Example 1) or the diagnosis device 10 (in the case of Configuration Example 2).

For example, the notification unit 110 may instruct the user 20 to have a first facial state or a second facial state. (Then, the facial state detection unit 102 may set the facial state instructed by the notification unit 110 as a detected facial state of the user.)

For example, when at least one of the first facial state and the second facial state is not detected within a certain period, the notification unit 110 may instruct the user 20 to have the facial state that has not been detected. The certain period may be set by the user 20 or may be determined based on the past detection time (for example, an average value, a longest value, or the like of the time required to detect the facial state of a plurality of users in the past, or a detection time set for each individual based on the past detection time for the each individual).

The diagnosis device 10 may skip (that is, no diagnosis process is performed) the process relating to the facial state that has not been detected within the certain period. The diagnosis device 10 may inquire the user 20 about the propriety of the skip. The diagnosis device 10 may switch between automatically executing the skip and inquiring the user 20 about the propriety of the skip. The diagnosis device 10 may notify the user 20 that the skip has been executed.

### <<Facial State>>

Here, the facial state will be described in detail. In the present specification, the case where the facial state of the user 20 is "facial expression" is mainly described, but the present invention is not limited thereto, and the facial state of the user 20 may be any facial state such as "face position", "face orientation", or the like. The facial state may be a combination of two or more of "facial expression", "face position", and "face orientation".

### [Facial Expression]

For example, the facial state of the user 20 is the facial expression of the user 20. In the present specification, the case where the facial expression is "straight face" and "smiling face" is mainly described, but the present invention is not limited thereto, and other facial expressions may be used.

The straight face is the facial expression when no emotion is presented on the face (no facial expression). For example, it is determined whether the user 20 has the straight face based on the coordinates of each part (for example, eyes, nose, mouth, and the like) of the face of the user 20 in the image. For example, when the height of the corners of the mouth is a certain value or less with respect to the height of the center of the mouth, it is determined to be the straight face. The determination may be made based on the result of machine learning that distinguishes between straight faces and smiling faces from a large number of photographs of facial expressions acquired in advance.

The smiling face is the facial expression of smiling. For example, it is determined whether the user 20 has the smiling face based on the coordinates of each part (for example, eyes, nose, mouth, and the like) of the face of the user 20 in the image. For example, when the height of the corners of the mouth is a certain value or more with respect to the height of the center of the mouth, it is determined to be the smiling face. The determination may be made based on the result of machine learning that distinguishes between straight faces and smiling faces from a large number of photographs of facial expressions acquired in advance.

### [Face Position]

For example, the facial state of the user 20 is the face position of the user 20.

For example, in the case where the facial state of the user 20 is "face position", when the distance between the face position of the user 20 and the position of the terminal 11 (in the case of Configuration Example 1) or the position of the diagnosis device 10 (in the case of Configuration Example 2) is short (in other words, when the face is being captured from close up), the diagnosis suitable for using an image of a face captured from close up may be performed, and when the distance between the face position of the user 20 and the position of the terminal 11 (in the case of Configuration Example 1) or the position of the diagnosis device 10 (in the case of Configuration Example 2) is long (in other words, when the face is being captured from a distance), the diagnosis suitable for using an image of a face captured from a distance may be performed.

For example, in the case where the facial state of the user 20 is "face position", when the facial state of the user 20 is captured in a first position, the diagnosis suitable for using an image of a face captured in the first position may be performed, and when the facial state of the user 20 is captured in a second position, the diagnosis suitable for using an image of a face captured in the second position may be performed.

### [Face Orientation]

For example, the facial state of the user 20 is the face orientation of the user 20.

For example, in the case where the facial state of the user 20 is "face orientation", when the facial state of the user 20 is captured in a first orientation, the diagnosis suitable for using an image of a face captured in the first orientation may be performed, and when the facial state of the user 20 is captured in a second orientation, the diagnosis suitable for using an image of a face captured in the second orientation may be performed.

### <<Diagnosis>>

Here, the diagnosis will be described in detail. In the present specification, the case where the diagnosis is the skin diagnosis and the stress diagnosis is mainly described, but the present invention is not limited thereto, and the diagnosis may be any diagnosis such as a physical diagnosis of a body including a face, a psychological or mental health diagnosis, and the like.

The image capturing method required for each diagnosis process is different. (That is, the imaging method in the first diagnosis process and the imaging method in the second diagnosis process are different.) The imaging method includes at least one of resolution, angle of view, focal length, and shutter speed.

### <<Example of Diagnosis>>

Examples of diagnosis will be described below.

· For example, in the skin diagnosis, tiny wrinkles may be identified from the frequency components of images of each part of the face.
· For example, in the skin diagnosis, length of the nasolabial fold may be detected by detecting the length of the edge component in the range below the cheek.
· For example, in the skin diagnosis, smoothness may be determined from the frequency components of an image of the entire cheek.
· For example, in the skin diagnosis, pores and clogged pores may be identified from the change in the luminance value and the magnitude of the change.
· For example, in the skin diagnosis, it is possible to make predictions by machine learning using measurement results of other devices and visual evaluation results as ground-truth data.

For example, in the stress diagnosis, the positions of the corners of the mouth and the outer corners of the eyes during the smiling face are detected, and the distortion of the face is detected from the positions of the corners of the mouth and the outer corners of the eyes on the left and right sides of the face. The degree of stress may be determined from the magnitude of the distortion of the face.

The following indicates the image capturing methods required for the skin diagnosis and the stress diagnosis.

### [Skin Diagnosis]

· Image resolution: 2,048×1,536 pixels
· Focus: Shallow field. To make it easier to detect unevenness of the skin.
· Image processing: Weak. To capture the delicate condition of the skin.
· Image processing: No edge processing. To capture the delicate condition of the skin.

### [Stress Diagnosis]

· Image resolution: 640×480 pixels
· Focus: Deep field (pan focus).
· Image processing: Strong. To make it easier to extract the coordinates of the outer corners of the eyes, and the like.
· Image processing: With edge processing. To make it easier to extract the coordinates of the outer corners of the eyes, and the like.

In addition, for example, the presence or absence of uneven chewing may be diagnosed using an image in which the facial state (the facial expression) is the straight face. For example, the presence or absence of teeth grinding or clenching may be diagnosed using an image in which the facial state (the facial expression) is the straight face. For example, sagging may be diagnosed using an image in which the facial state (the face orientation) is sideways. For example, the scalp may be diagnosed using an image in which the facial state (the face orientation) is downward. For example, the scalp may be diagnosed using an image in which the facial state (the face position) is lower relative to the camera lens.

### <Method>

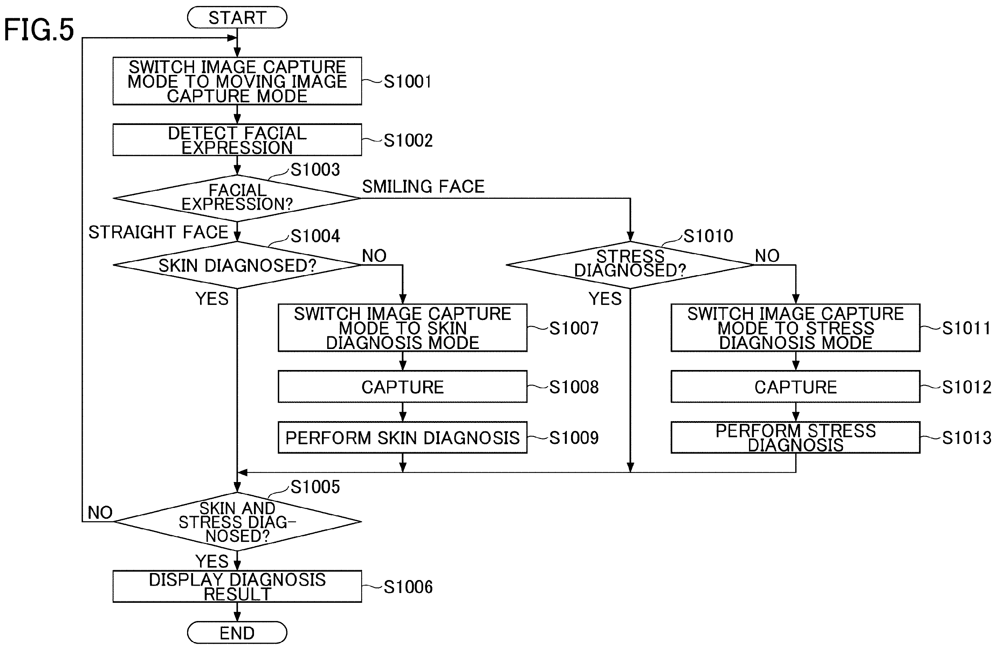

FIG. 5 is a flowchart of a diagnosis process according to an embodiment of the present invention. When it is detected that the user 20 is in front of the terminal 11 (in the case of Configuration Example 1 in FIG. 1) or the diagnosis device 10 (in the case of Configuration Example 2 in FIG. 1), or when the user 20 inputs an instruction to the terminal 11 (in the case of Configuration Example 1 in FIG. 1) or the diagnosis device 10 (in the case of Configuration Example 2 in FIG. 1), the process starts.

In step 1001 (S1001), the diagnosis device 10 switches the image capture mode of the terminal 11 (in the case of Configuration Example 1 in FIG. 1) or the diagnosis device 10 (in the case of Configuration Example 2 in FIG. 1) to "moving image capture mode". The image captured in "moving image capture mode" may be of such a quality that the facial expression of the user 20 can be detected.

In step 1002 (S1002), the diagnosis device 10 detects the facial expression of the user 20 while capturing the moving image in "moving image capture mode" switched in S1001.

In step 1003 (S1003), the diagnosis device 10 determines whether the facial expression detected in S1002 is "straight face" or "smiling face". When the facial expression is "straight face", the process proceeds to step 1004, and when the facial expression is "smiling face", the process proceeds to step 1010.

In step 1004 (S1004), the diagnosis device 10 determines whether the diagnosis using an image of a straight face (the skin diagnosis) has been completed. When the skin diagnosis has been completed, the process proceeds to step 1005, and when the skin diagnosis has not been completed, the process proceeds to step 1007.

In step 1005 (S1005), the diagnosis device 10 determines whether both the skin diagnosis and the stress diagnosis have been completed. When both the skin diagnosis and the stress diagnosis have been completed, the process proceeds to step 1006, and when either the skin diagnosis or the stress diagnosis has not been completed, the process returns to S1001.

In step 1006 (S1006), the diagnosis device 10 causes the terminal 11 (in the case of Configuration Example 1 in FIG. 1) or the diagnosis device 10 (in the case of Configuration Example 2 in FIG. 1) to display the diagnosis result.

In step 1007 (S1007), the diagnosis device 10 switches the image capture mode of the terminal 11 (in the case of Configuration Example 1 in FIG. 1) or the diagnosis device 10 (in the case of Configuration Example 2 in FIG. 1) to "skin diagnosis mode".

In step 1008 (S1008), the diagnosis device 10 causes the terminal 11 (in the case of Configuration Example 1 in FIG. 1) or the diagnosis device 10 (in the case of Configuration Example 2 in FIG. 1) to capture a still image with high quality.

In step 1009 (S1009), the diagnosis device 10 performs the skin diagnosis process using the highquality still image of the straight face captured in S1008. Then, the process proceeds to step 1005.

In step 1010 (S1010), the diagnosis device 10 determines whether the diagnosis using an image of a smiling face (the stress diagnosis) has been completed. When the stress diagnosis has been completed, the process proceeds to step 1005, and when the stress diagnosis has not been completed, the process proceeds to step 1011.

In step 1011 (S1011), the diagnosis device 10 switches the image capture mode of the terminal 11 (in the case of Configuration Example 1 in FIG. 1) or the diagnosis device 10 (in the case of Configuration Example 2 in FIG. 1) to "stress diagnosis mode".

In step 1012 (S1012), the diagnosis device 10 causes the terminal 11 (in the case of Configuration Example 1 in FIG. 1) or the diagnosis device 10 (in the case of Configuration Example 2 in FIG. 1) to capture a still image with low quality.

In step 1013 (S1013), the diagnosis device 10 performs the stress diagnosis process using the lowquality still image of the smiling face captured in S1012. Then, the process proceeds to step 1005.

### <Effect>

As described above, in an embodiment of the present invention, because the process is switched to the diagnosis (for example, the skin diagnosis or the stress diagnosis) process according to the facial state (for example, the facial expression, the face position, the face orientation, and the like) of the user, multiple types of diagnoses may be easily performed without inconveniencing the user, the beauty staff, or the like.

### <Hardware Configuration>

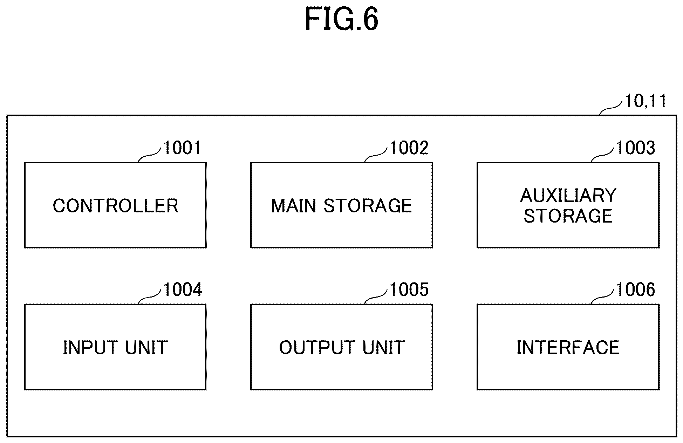

FIG. 6 is a hardware configuration diagram of the diagnosis device 10 and the terminal 11 according to an embodiment of the present invention. The diagnosis device 10 and the terminal 11 may include a controller 1001, a main storage 1002, an auxiliary storage 1003, an input unit 1004, an output unit 1005, and an interface 1006. Each of these components will be described below.

The controller 1001 is a processor (for example, central processing unit (CPU), graphics processing unit (GPU), and the like) that executes various programs installed in the auxiliary storage 1003.

The main storage 1002 includes a nonvolatile memory (read only memory (ROM)) and a volatile memory (random access memory (RAM)). The ROM stores various programs, data, and the like necessary for the controller 1001 to execute the various programs installed in the auxiliary storage 1003. The RAM provides a working area into which the various programs installed in the auxiliary storage 1003 are expanded when executed by the controller 1001.

The auxiliary storage 1003 is an auxiliary storage device that stores various programs and information used when the various programs are executed.

The input unit 1004 is an input device through which an operator of the diagnosis device 10 and the terminal 11 inputs various instructions to the diagnosis device 10 and the terminal 11.

The output unit 1005 is an output device that outputs the internal states and the like of the diagnosis device 10 and the terminal 11.

The interface 1006 is a communication device for connecting to a network and communicating with other devices.

Although the embodiments of the present invention have been described in detail above, the present invention is not limited to the specific embodiments described above, and various changes and modifications can be made within the scope of the present invention described in the claims.

The present international application claims priority to Japanese Patent Application No. 2022-171404 filed October 26, 2022, the entire contents of which are incorporated herein by reference.

### DESCRIPTION OF REFERENCE NUMERALS

1 Diagnosis system
10 Diagnosis device
11 Terminal
20 User
101 Image acquisition unit
102 Facial state detection unit
103 First determination unit
104 Second determination unit
105 Diagnosis process switching unit
106 First diagnosis processing unit
107 Second diagnosis processing unit
108 Diagnosis history storage
109 Reception unit
110 Notification unit
1001 Controller
1002 Main storage
1003 Auxiliary storage
1004 Input unit
1005 Output unit
1006 Interface

## Claims

1. A diagnosis system that includes a terminal and a server, the system comprising:
a first diagnosis processing unit configured to perform a first diagnosis process;
a second diagnosis processing unit configured to perform a second diagnosis process different from the first diagnosis process;
a facial state detection unit configured to detect a facial state of a user; and
a diagnosis process switching unit configured to determine which of the first diagnosis process or the second diagnosis process is to be performed according to a result of the detecting.

2. The diagnosis system according to claim 1, wherein the facial state is a facial expression.

3. The diagnosis system according to claim 2, wherein the facial state detection unit is configured to detect the facial expression of the user based on features of an image of a face of the user captured by the terminal.

4. The diagnosis system according to claim 1, wherein the first diagnosis process and the second diagnosis process differ in an imaging method.

5. The diagnosis system according to claim 4, wherein the imaging method is a resolution of an image used for a diagnosis.

6. The diagnosis system according to claim **1,** further comprising:
a diagnosis history storage configured to store a history of performing the first diagnosis process and a history of performing the second diagnosis process;
a first determination unit configured to determine whether the first diagnosis process is performed a predetermined number of times in a session; and
a second determination unit configured to determine whether the second diagnosis process is performed a predetermined number of times in the session, wherein
the first diagnosis processing unit is configured to perform the first diagnosis process when the first diagnosis process is not performed the predetermined number of times, and
the second diagnosis processing unit is configured to perform the second diagnosis process when the second diagnosis process is not performed the predetermined number of times.

7. The diagnosis system according to claim **1,** further comprising:
a diagnosis history storage configured to store a history of performing the first diagnosis process and a history of performing the second diagnosis process;
a first determination unit configured to determine whether the first diagnosis process is performed a predetermined number of times outside a session; and
a second determination unit configured to determine whether the second diagnosis process is performed a predetermined number of times outside the session, wherein
the first diagnosis processing unit is configured to perform the first diagnosis process when the first diagnosis process is not performed the predetermined number of times, and
the second diagnosis processing unit is configured to perform the second diagnosis process when the second diagnosis process is not performed the predetermined number of times.

8. The diagnosis system according to claim 6 or 7, wherein a setting of the predetermined number of times by the user is received.

9. The diagnosis system according to claim 6 or 7, wherein the history of performing the first diagnosis process and the history of performing the second diagnosis process have expiration dates set.

10. The diagnosis system according to claim **1,** wherein a selection of only the first diagnosis process, only the second diagnosis process, or both the first diagnosis process and the second diagnosis process by the user is received, and the received diagnosis process is performed.

11. The diagnosis system according to claim **1,** further comprising:
a notification unit configured to instruct the user to have a first facial state or a second facial state, wherein
the facial state detection unit is configured to set the facial state instructed by the notification unit as a detected facial state of the user.

12. The diagnosis system according to claim 1, further comprising:
a notification unit configured to instruct, when at least one of a first facial state and a second facial state is not detected within a certain period of time, the user to have the facial state that is not detected.

13. The diagnosis system according to claim 12, wherein the certain period of time is set by the user or determined based on a past detection time.

14. The diagnosis system according to claim 12, wherein a process relating to the facial state that is not detected within the certain period of time is skipped.

15. The diagnosis system according to claim 14, wherein a propriety of a skip is queried to the user.

16. The diagnosis system according to claim 14, wherein switching is made between automatically executing a skip and querying a propriety of a skip to the user.

17. The diagnosis system according to claim 14, wherein a notification is given to the user that a skip is executed.

18. The diagnosis system according to claim 1, wherein a process is started when it is detected that the user is in front of the terminal or when the user inputs an instruction to the terminal.

19. A diagnosis device comprising:
a first diagnosis processing unit configured to perform a first diagnosis process;
a second diagnosis processing unit configured to perform a second diagnosis process different from the first diagnosis process;
a facial state detection unit configured to detect a facial state of a user; and
a diagnosis process switching unit configured to determine which of the first diagnosis process or the second diagnosis process is to be performed according to a result of the detecting.

20. A program for causing a computer to function as:
a first diagnosis processing unit configured to perform a first diagnosis process;
a second diagnosis processing unit configured to perform a second diagnosis process different from the first diagnosis process;
a facial state detection unit configured to detect a facial state of a user; and
a diagnosis process switching unit configured to determine which of the first diagnosis process or the second diagnosis process is to be performed according to a result of the detecting.

21. A diagnosis method performed by a diagnosis device, the method comprising:
a step of detecting a facial state of a user;
a step of determining which of a first diagnosis process or a second diagnosis process different from the first diagnosis process is to be performed according to a result of the detecting; and
a step of performing the first diagnosis process or the second diagnosis process.

22. A skin diagnosis method performed by a diagnosis device, the method comprising:
a step of detecting that a facial expression of a user is a straight face;
a step of capturing a face of the user with high resolution in response to detecting the straight face; and
a step of diagnosing a skin of the user by using a high-resolution image in which the straight face of the user is captured.

23. A stress diagnosis method performed by a diagnosis device, the method comprising:
a step of detecting that a facial expression of a user is a smiling face;
a step of capturing a face of the user with low resolution in response to detecting the smiling face; and
a step of diagnosing a stress of the user by using a low-resolution image in which the smiling face of the user is captured.
